Europäisches Patentamt

European Patent Office

Office européen des brevets

(11) Veröffentlichungsnummer: **0 167 727**
A2

(12) # EUROPÄISCHE PATENTANMELDUNG

(21) Anmeldenummer: **85104638.3**

(22) Anmeldetag: **17.04.85**

(51) Int. Cl.⁴: **A 61 F 2/64**

(30) Priorität: **19.04.84 DE 3414869**

(43) Veröffentlichungstag der Anmeldung:
**15.01.86 Patentblatt 86/3**

(84) Benannte Vertragsstaaten:
**CH DE FR LI**

(71) Anmelder: **Teufel, Wilhelm Julius**
**Neckarstrasse 189 - 191**
**D-7000 Stuttgart 1(DE)**

(72) Erfinder: **Amrein, Max**
**Chaussée de la Roche**
**CH-1020 Renens(CH)**

(74) Vertreter: **Maier, Eugen, Dr.-Ing. et al,**
**Patentanwälte Dr.-Ing. Eugen Maier Dr.-Ing. Eckhard**
**Wolf, Dr.-Ing. H.J.Vetter Pischekstrasse 19**
**D-7000 Stuttgart 1(DE)**

(54) **Künstliches Kniegelenk.**

(57) Das Kniegelenk gemäß der Erfindung weist gegenüber bekannten künstlichen Kniegelenken, die je ein vorderes und ein hinteres, durch einen Lenker verbundenes Drehgelenk aufweisen, eine einfachere Konstruktion auf, die darin besteht, daß in dem unteren Gelenkteil (1) ein federbelasteter, in Längrichtung dieses Gelenkteils beweglicher Sperrbolzen (14) angeordnet ist, der bei abgewinkeltem Gelenk gegen die Umfangsfläche eines einen Teil des oberen Gelenks (2) bildenden Kurvenstücks (22) anliegt, das an den Schenkeln des gabelförmig ausgebildeten oberen Endes des unteren Gelenkteils (1) angelenkt ist und das in der Sperrstellung bei im wesentlichen miteinander fluchtenden Gelenkteilen von dem an seinem oberen Ende eine Schrägfläche aufweisenden Sperrbolzen (14) hintergriffen wird.

Fig. 2

EP 0 167 727 A2

- 1 -

0167727

Beschreibung

Die Erfindung betrifft ein künstliches Kniegelenk der im Oberbegriff des Anspruchs 1 angegebenen Gattung. Ein Kniegelenk der vorgenannten Gattung ist aus DE-PS 188 194 bekannt, bei dem in das untere Gelenkteil ein mit dem Fußteil einstückig verbundener Zapfen hineinragt, dessen oberes Ende als Sperrbolzen ausgebildet ist, dessen abgerundete Stirnfläche in eine Ausnehmung des oberen Gelenkteils bei durch das Körpergewicht belastetem Bein einrastbar ist. Das Einrasten wird dadurch bewirkt, daß das obere Gelenkteil zusammen mit dem unteren Gelenkteil entgegen der Federkraft der am Umfang des Sperrbolzens angeordneten Schraubenfeder in Richtung auf das Fußteil bewegt wird, bis das untere Gelenkteil auf dem Fußteil aufsitzt.

Dieses Kniegelenk weist den Nachteil auf, daß eine Verrastung des oberen mit dem unteren Gelenkteil nur bei belastetem Bein erfolgt, was sich beim Gehen nachteilig auswirkt.

Demgegenüber liegt der Erfindung die Aufgabe zugrunde, ein künstliches Kniegelenk so auszubilden, daß die Verrastung der beiden Gelenkteile nur durch Betätigung eines auf den Sperrbolzen einwirkenden Organs bewirkt werden kann.

Ein künstliches Kniegelenk, bei dem die Verrastung der beiden Gelenkteile ebenfalls nur mittels eines von Hand betätigten Organs aufgehoben wird, ist aus der DE-PS 28 41 999 bekannt. Dieses Kniegelenk weist je ein vorderes und ein hinteres Drehgelenk auf, von denen jeweils die vorderen und die hinteren Drehgelenke durch einen Lenker miteinander verbunden sind. Die Konstruktion dieses Kniegelenks erfordert eine aufwendige Montage und hat darüber hinaus den Nachteil, daß die Auslösung der das Kniegelenk bei in einer gestreckten Lage befindlichen Gelenkteilen verrastenden Sperre durch eine nach hinten gerichtete, an dem Sperrglied angreifende Zugkraft erfolgt, wodurch auf das Kniegelenk als ganzes ein die Befestigung des Kniegelenks am Oberschenkel beeinträchtigendes Drehmoment ausgeübt wird.

Diesem Kniegelenk gegenüber sieht die Erfindung eine wesentlich einfachere Konstruktion vor, die ein weicheres Auslösen der Sperre und das Auslösen der Sperre durch Zug an dem auf das Sperrglied einwirkenden Organ in Richtung auf den mit der Unterschenkelprothese fluchtenden Oberschenkel erlaubt.

Ein weiterer Vorteil des erfindungsgemäßen Kniegelenks besteht darin, daß das das Auslösen der Sperre bewirkende Organ über die Außenseite der Prothese übersteht und das Auslösen somit an der jeweiligen, besser zugänglichen Außenseite des Kniegelenks erfolgen kann.

In der Zeichnung ist ein Ausführungsbeispiel des Kniegelenks in schematischer Weise dargestellt. Es zeigen

Fig. 1 eine Vorderansicht des Kniegelenks in der Sperrstellung;

Fig. 2 einen senkrechten Schnitt durch das Kniegelenk
nach der Schnittlinie 2 - 2 der Fig. 1;

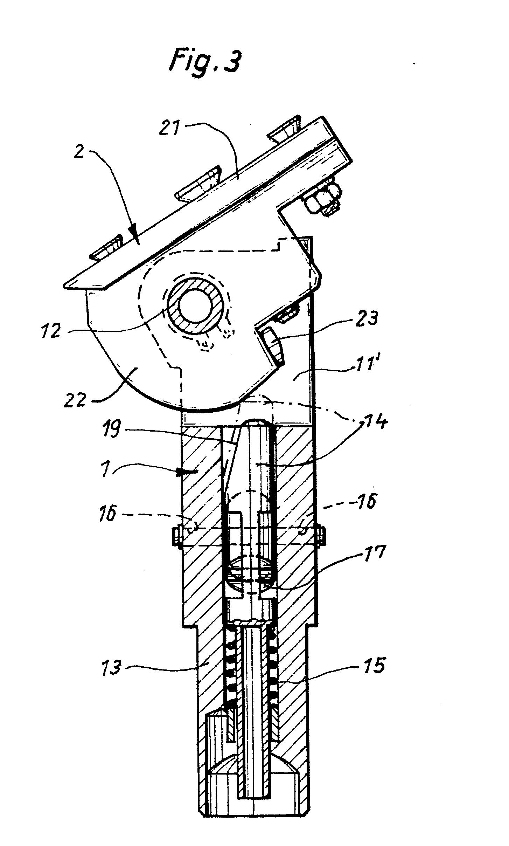

Fig. 3 einen der Fig. 2 entsprechenden Schnitt durch
das Kniegelenk bei abgewinkelter Unterschenkelprothese.

Das Gelenk besteht aus dem unteren Gelenkteil 1 und dem
oberen Gelenkteil 2, die mittels des Gelenkbolzens 12
schwenkbar miteinander verbunden sind. Das untere Gelenkteil 1 ist in seinem oberen Bereich gabelförmig ausgebildet und weist in seinen beiden Schenkeln 11 und 11'
dem Gelenkbolzen 12 als Lager dienende Bohrungen auf.
Im unteren Bereich des unteren Gelenkteils 1 ist eine
axiale Bohrung vorgesehen, in der der Sperrbolzen 14 geführt
ist. Dieser weist eine untere Ringschulter auf, gegen die
eine Schraubenfeder 15 anliegt, deren unteres Ende gegen
eine Hülse anliegt, die in das untere zylindrisch ausgebildete Ende 13 des unteren Gelenkteils 1 eingesetzt ist.
Dieses untere Ende 13 dient zur Verbindung des Kniegelenks
mit der Unterschenkelprothese. In seinem mittleren Bereich
weist das untere Gelenkteil 1 einen Durchbruch 18 zur
Aufnahme eines doppelarmigen Hebels 17 auf, der mittels
eines Bolzens schwenkbar in Bohrungen 16 gelagert ist.
Das Ende des mit dem Sperrbolzen 14 zusammenwirkenden
Lastarms des doppelarmigen Hebels 17 ist gabelförmig ausgebildet und umfaßt mit seinen Schenkeln den in seinem
mittleren Bereich verjüngt ausgebildeten, zwei parallele
Seitenflächen aufweisenden Sperrbolzen 14. In die endseitigen Schenkel des Lastarms des Hebels 17 ist ein den
verjüngten Teil des Sperrbolzens 14 durchsetzender Bolzen eingesetzt, der die Schwenkbewegung des Hebels 17 in eine Längsverschiebung des Sperrbolzens 14 umsetzt. Das obere Ende des eine ballig ausgebildete Stirnfläche aufweisenden Sperrbolzens 14 weist eine seitliche Schrägfläche auf, die ein Aufgleiten und ein Abgleiten des Sperrbolzens 14 bei der Verriegelung und der Entriegelung der beiden Gelenkteile 1 und 2 erleichtert.

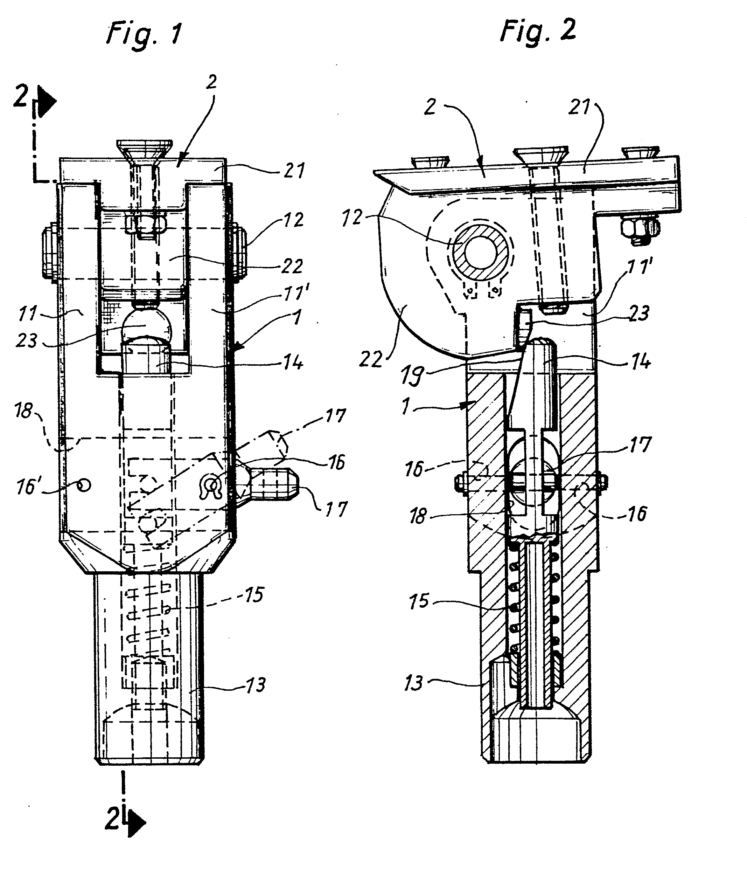

Das obere Gelenkteil 2 weist eine Platte 21 auf, die der Verbindung des Gelenkteils 2 mit einer den Oberschenkel umfassenden Bandage dient. An der Unterseite der Platte 21 ist ein Kurvenstück 22 angeformt, das in den von den beiden Schenkeln 11 und 11' gebildeten Zwischenraum eingreift und mit diesen beiden Schenkeln mittels des Gelenkbolzens 12 schwenkbar verbunden ist. Das Kurvenstück 22 weist eine gekrümmte Umfangsfläche auf, gegen die die ballig ausgebildete Stirnfläche des Sperrbolzens 14 während der Schwenkbewegung unter geringer Gleitreibung anliegt. In der in Fig. 1 und 2 dargestellten Verriegelungsstellung liegt der Sperrbolzen 14 mit seiner Schrägfläche gegen die Schrägfläche einer an dem Kurvenstück 22 angebrachten kleinen Stahlplatte 23 an.

Patentansprüche

1. Künstliches Kniegelenk für eine Prothese mit einem oberen und einem unteren Gelenkteil und einem federbelasteten, im unteren Gelenkteil längsbeweglich angeordneten, mit dem ein Kurvenstück aufweisenden, oberen Gelenkteil verrastbaren Sperrbolzen, d a - d u r c h  g e k e n n z e i c h n e t , daß in dem unteren Gelenkteil (1) ein doppelarmiger, mit seinem Kraftarm über das untere Gelenkteil (1) seitlich überstehender, am Sperrbolzen (14) angreifender Hebel (17) gelagert ist und und das Kurvenstück (22) am Ende seiner gekrümmten Umfangsfläche eine mit der Umfangsfläche einen Winkel einschließende Rastfläche aufweist, gegen die der Sperrbolzen (14) in der Sperrstellung anliegt.

2. Kniegelenk nach Anspruch 1,  d a d u r c h  g e - k e n n z e i c h n e t ,  daß das untere Gelenkteil (1) gabelförmig ausgebildet ist und der Sperrbolzen (14) zwischen die Schenkel (11,11') dieses Gelenkteils (1) eingreift.

3. Kniegelenk nach den Ansprüchen 1 und 2,  d a - d u r c h  g e k e n n z e i c h n e t ,  daß unterhalb der beiden Schenkel (11,11') des unteren Gelenkteils (1) je ein Lager (16,16') für den umsetzbaren doppelarmigen, den Sperrbolzen (14) betätigenden Hebel (17) angeordnet ist.

4. Kniegelenk nach den Ansprüchen 1 bis 3, d a -
d u r c h   g e k e n n z e i c h n e t , daß der
Sperrbolzen (14) eine an seine ballig ausgebildete
Stirnfläche angrenzende, in Richtung auf das Kurvenstücke (22) weisende Schrägfläche (19) aufweist.

5. Kniegelenk nach den Ansprüchen 1 bis 4, d a -
d u r c h   g e k e n n z e i c h n e t ,   daß
das Kurvenstück (22) zwischen seiner gekrümmten
Umfangsfläche und der dem Sperrbolzen (14) in der
Sperrstellung als Anlage dienenden Rastfläche einen
abgerundeten Bereich aufweist.

6. Kniegelenk nach einem der Ansprüche 1 bis 5, d a -
d u r c h   g e k e n n z e i c h n e t ,   daß
die Rastfläche des Kurvenstücks (22) eine Stahlauflage (23) aufweist.

0167727

112

Fig. 1

Fig. 2

## Fig.3